# EUROPEAN PATENT APPLICATION

(11) **EP 4 752 899 A1**
(43) Date of publication of application: **03.06.2026**
(21) Application number: 24216031.5
(22) Date of filing: 28.11.2024
(51) Int. Cl.: G16H 30/20, G16H 40/63, G16H 40/67

(54) **MONITORING OF A REMOTE MEDICAL IMAGING CONTROL SYSTEM**

(71) Applicant: Koninklijke Philips N.V., 5656 AE Eindhoven (NL)
(72) Inventor: CHALUVANAHALLI RUDRAPPA, Deepak, Eindhoven (NL); RAMASAMY, Jaikumar, Eindhoven (NL); CHANDRASHEKARA, Manohar, 5656AG Eindhoven (NL); SUMAN, Swarnim, Eindhoven (NL); CHILLAPALLI, Jyothi, Eindhoven (NL); CHOKKALINGAM, Prakash, Eindhoven (NL)
(74) Representative: Philips Intellectual Property & Standards

(57) **Abstract**

Disclosed herein is a computer-implemented method of operating a remote medical imaging control system (300, 400, 500) that comprises at least one medical imaging node (302) with a medical imaging system (310) and a local controller (312). The at least one medical imaging node comprises a node specific control interface (314) with a screen replicator (404) and an input peripheral (406). The remote medical imaging control system further comprises at least one control node (304, 306). The method comprises: repeatedly monitoring (200) the at least one medical imaging node and repeatedly updating (202) its operational status (122) in a system status database (124); repeatedly monitoring (204) the at least one control node and updating (206) its the operational status (126) in the system status database; and providing (208) a system status (128) of the remote medical imaging control system using the system status database.

## Description

### FIELD OF THE INVENTION

The invention relates to medical imaging, in particular to communication systems which enable the remote access of medical imaging systems.

### BACKGROUND OF THE INVENTION

Various types of medical imaging systems such as magnetic resonance imaging systems, computer tomography systems, or other tomographic medical imaging systems are complicated and difficult to operate. To assist operators and ensure efficient operation of medical imaging systems they may be accessed remotely. A remote operator may have remote desktop control or access to multiple remote medical imaging systems simultaneously. In addition to having remote desktop control there may be additional audio and/or video feeds which enable the remote operator to interact with local operators of the multiple remote medical imaging systems.

### SUMMARY OF THE INVENTION

The invention provides for a computer-implemented method, a computer program, and a status tracking system in the independent claims. Embodiments are given in the dependent claims.

In one aspect a computer-implemented method of operating a remote medical imaging control system is disclosed. The remote medical imaging control system comprises at least one medical imaging node comprising a medical imaging system and a local controller. The local controller is configured for controlling image acquisition of the medical imaging system. The at least one medical imaging node comprises a node-specific controller interface. The node-specific controller interface comprises a screen replicator configured to provide a replication of a user interface of the local controller via a network connection. The node-specific controller interface further comprises at least one peripheral configured for receiving imaging control signals via the network interface. The imaging control signals are configured for controlling the user interface.

The remote medical imaging control system further comprises at least one control node configured for connecting to the node-specific controller interface via the network connection. The at least one control node is further configured to provide the imaging control signals via the network connection in response to receiving the replicated user interface via the network connection.

The method comprises repeatedly monitoring the at least one medical imaging node to determine an operational status of the at least one node-specific controller interface. The method further comprises repeatedly updating the operational status of the at least one node-specific controller interface in a system status database. The method further comprises repeatedly monitoring the at least one control node to determine an operational status of the at least one control node. The method further comprises repeatedly updating the operational status of the at least one control node in the system status database. The method further comprises providing a system status descriptive of an operational status of the remote medical imaging control system using the system status database.

In another aspect, a computer program product comprising a computer-readable storage medium having machine executable instructions embodied therewith. The machine executable instructions are configured to implement and example of the method.

In another aspect, a status tracking system is disclosed. The status tracking system comprises a processor. The status tracking system further comprises a memory storing machine-executable instructions. The execution of the machine-executable instructions causes the processor to repeatedly monitor the at least one medical imaging node to determine an operational status of at least one node-specific controller interface. The at least one medical imaging node comprises a medical imaging system and a local controller. The local controller is configured for controlling image acquisition of the medical imaging system. The at least one medical imaging node comprises the node-specific controller interface. The node-specific controller interface comprises a screen replicator configured to provide replication of a user interface of the local controller via a network connection. The node-specific controller interface further comprises at least one input peripheral configured for receiving imaging control signals via the network interface. The imaging control signals are configured for controlling the user interface. Execution of the machine-executable instructions further causes the processor to repeatedly update the operational status of the at least one node-specific controller interface in a system status database. The at least one control node is configured for connecting to the node-specific controller interface via the network connection. The at least one control node is further configured to provide the imaging control signals via the network connection in response to receiving the replicated user interface via the network connection. Execution of the machine-executable instructions further causes the processor to repeatedly update the operational status of the at least one node-specific controller interface in a system status database. The at least one control node is configured for connecting to the node-specific controller interface via the network connection. The at least one control node is further configured to provide the image control signals via the network connection in response to receiving the replicated user interface via the network connection.

Execution of the machine-executable instructions further causes the process to repeatedly monitor the at least one control node to determine the operational status of the at least one control node. Execution of the machine-executable instructions further causes the processor to repeatedly update the operational status of the at least one control node in the system status database. Execution of the machine-executable instructions further causes the processor to provide a system status descriptive of an operational status of the remote medical imaging control system using the system status database.

### BRIEF DESCRIPTION OF THE DRAWINGS

In the following preferred embodiments of the invention will be described, by way of example only, and with reference to the drawings in which:
- Fig. 1: illustrates an example of a status tracking system.
- Fig. 2: shows a flow chart which illustrates a method of using the status tracking system of Fig. 1.
- Fig. 3: illustrates an example of a remote medical imaging control system.
- Fig. 4: illustrates a further example of a remote medical imaging control system.
- Fig. 5: illustrates a further example of a remote medical imaging control system.

### DETAILED DESCRIPTION OF EMBODIMENTS

Like numbered elements in these figures are either equivalent elements or perform the same function. Elements which have been discussed previously will not necessarily be discussed in later figures if the function is equivalent.

Examples may be beneficial because it provides for an effective means of providing a real time system status that is descriptive of the operational status of the remote medical imaging control system (or ROCC Remote Operations Control Center). The repeated monitoring and updating of the operational status in the system status database provides an effective means for having up to date information about which components are active and able to function properly. This may for example be highly beneficial in an emergency system when it is needed to provide supervision or operation of the medical imaging system remotely. In another aspect, a computer program product comprising a computer-readable storage medium having machine-executable instructions embodied therewith is disclosed. The machine-executable instructions are configured to implement an example of the method.

In one aspect a computer-implemented method of operating the remote medical imaging control system is disclosed. A remote medical imaging control system may encompass various components and systems which enable the remote control of a medical imaging system. For example, a user interface could be provided to a remote operator which may be used to operate medical imaging systems such as a computed tomography or a magnetic resonance imaging system. The advantages of the system is that it may provide a means of providing expertise to various locations when it is not possible to have skilled technologists or radiologists on call or available all the time.

The remote medical imaging control system comprises at least one medical imaging node comprising a medical imaging system and a local controller. The medical imaging system may be various modalities of medical imaging systems such as a computed tomography system, a magnetic resonance imaging system, a diagnostic ultrasound system, a single photon emission tomography system, a positron emission tomography system, a digital X-ray machine, and/or a digital fluoroscopy machine. The local controller may be a computer or microcontroller which contains instructions which are used to operate and control the medical imaging system. The local controller is configured for controlling image acquisition of the medical imaging system. The at least one medical imaging node comprises a node-specific controller interface.

The node-specific controller interface in this example comprises a screen replicator configured to provide a replication of a user interface of the local controller via a network connection. The node-specific controller interface further comprises at least one input peripheral configured for receiving imaging control signals via the network interface. The imaging control signals are configured for controlling the user interface.

The node-specific controller interface may be implemented in different ways in different examples. In one example, the node-specific controller interface may for example be a software component which enables remote control of the local controller remotely. In other examples, the node-specific controller interface may be a hardware component. For example, the monitor cable may be plugged into the node-specific controller interface and then the screen is then replicated or provided over the internet. Likewise, the node-specific controller interface may provide a USB or other interface which is plugged into the machine and replicates a human user interface device such as a mouse or keyboard. The use of a separate hardware interface component may be beneficial in many situations because it is not necessary and prevents the installation of additional software or altering the software on a local controller. This may be particularly beneficial when it is unavailable or undesirable to modify the software.

The remote medical imaging control system further comprises at least one control node that is configured for connecting to the node-specific controller interface via the network connection. The at least one control node may for example be at least one computer system or computational system that is able to provide commands for remote control of the local controller. The at least one control node is further configured to provide the imaging control signals via the network connection in response to receiving the replicated user interface via the network connection.

The method comprises repeatedly monitoring the at least one medical imaging node to determine an operational status of the at least one node-specific controller interface. The method further comprises repeatedly updating the operational status of the at least one node-specific controller interface in a system status database. The system status database as used herein may encompass various types of storage systems or databases. In one example, the system status database is a relational database. In other examples, the system status database could be a file or folder which contains information which is stored about the remote medical imaging control system. For example, instead of a database, the information could be stored in a formatted xml file. The method further comprises repeatedly monitoring the at least one control node to determine an operational status of the at least one control node.

The method further comprises repeatedly updating the operational status of the at least one control node in the system status database. The method further comprises providing a system status descriptive of an operational status of the remote medical imaging control system using the system status database. The providing the system status may take different forms in different examples. In one example the system status could provide a table or summary of the various components or some of the components of the remote medical imaging control system. In another example the system status could be a status of a particular component in response to a query. In further examples the system status could be provided in response to searching for a functional component, for example in an emergency situation.

In various examples different means may be used for monitoring
In a further example, the method further comprises receiving an alert signal from the local controller of the medical imaging system. For example, the alert could have been provided manually or automatically. For example, the alert signal could be a request from a person operating the local controller. In other examples, an automatic alarm such as a fire alarm, a warning for a quenching of an MRI system or other emergency could automatically trigger the generation of the alert signal. The method further comprises searching the system status database for an operational control node. The at least one control node comprises the operational control node. The control nodes provide the remote operator the ability to operate and control the medical imaging system via the local controller.

By searching the system database for an operational control node a functional system is then found using the up to date or near up to date system status in the system status database. The method further comprises forming the network connection between the operational control node and the at least one medical imaging node. The method further comprises sending the replicated user interface from the local controller to the operational control node via the network connection. The method further comprises sending the imaging control signals from the operational control node to the node-specific controller interface via the network connection in response to receiving the replicated user interface. This example may be advantageous because in response to the alert signal the operational control node is automatically found and the connection is established. This for example may reduce the delay in finding an operational control node when there is an emergency situation or problem with the local controller or the medical imaging system itself. This may for example provide for improved safety and also may result in more efficient use of the medical imaging system.

In another example, the operational control node comprises a robotic process automation system configured to generate the imaging control signals in response to receiving the replicated user interface and the alert signal. As opposed to an operator providing the imaging control signals, it is provided by a robotic process automation system.

Robotic Process Automation (RPA) uses software robots (bots) to automate repetitive or rule-based tasks. These bots can mimic human interactions with computer systems by controlling human interface devices such as keyboards and mice. As they may work using rules, software bots could be programmed to respond in a particular way to a given alert signal.

For example, the alert signal may contain specific details about the type of alert or problem with the local controller and/or the medical imaging system. In response to the specific problem the robotic process automation system (bot) may for example call up a script which is used to automatically deal with or provide more information on the problem.

In another example, the remote medical imaging control system further comprises at least one hospital zone. The at least one hospital zone comprises multiple of the at least one medical imaging node. The repeated monitoring of the at least one medical imaging node is performed locally with the at least one hospital zone. The method further comprises pulling the at least one hospital zone to update the operational status of the at least one medical imaging node in the system status database. As opposed to connecting with all of the individual components of the remote medical imaging itself, the data may first be pooled locally in a particular hospital zone and then either pushed or pulled to be included in the system status database. This may have the benefit of also providing local information on the performance of the system as well as providing it globally via the system status database.

In another example, the local monitoring of the at least one medical imaging node is performed using a KVM manager controlled by a console service. A KVM manager is a system to monitor the operation of various KVM systems. A KVM system is a system which provides the remote keyboard, video and mouse to the local controller. This is an example of a type of node-specific controller interface. The use of the console service may for example provide an easy to implement and cost-effective means of providing this locally within the hospital zone.

In some of these examples the console service is preferably scheduled using a cron job. This may for example be beneficial because the system can be set up to run in an automated fashion.

In another example, the node-specific controller interface is further configured to provide a video feed via the network connection. The operational status of the node-specific controller interface is descriptive of an operational status of the video feed. The video feed is any one of the following: a medical imaging node controller and video feed and a medical imaging node imaging system room video feed and combinations thereof. In these remote systems it may be beneficial for example to provide a video which shows the subject in an examination room or also providing a video of what is happening in the control room for the medical imaging system.

In another example, the node-specific controller interface is further configured to provide a bidirectional intercom channel via the network connection. The bidirectional intercom channel may for example be between the local operator and the subject in the medical imaging system. In other examples, the bidirectional intercom channel may be between the local and the remote operators. In other further examples, the bidirectional intercom may be interfaced in such a way that it is between the subject being imaged in the medical imaging system and either/or the local or the remote operators. Providing the status of the intercom system may be beneficial in ensuring that the system is functioning properly and that the operator is able to help the local operator or subject being imaged in an efficient and safe manner.

In another example, the remote medical imaging control system comprises a monitoring service configured for monitoring the at least one control node. For example, the monitoring service may be provided on a remote machine or in the cloud and may provide services to the remote medical imaging control system globally. This may be beneficial as the system status database may for example be hosted independently of the other components of the remote medical imaging control system.

In another example, the remote medical imaging control system comprises a platform monitor that hosts the system status database. In this example, there is a platform monitor which provides the system status database as a separate component or location and may therefore be more easily accessible and available to various components or operators of the remote medical imaging control system.

In another example, the monitoring of the at least one medical imaging node and/or the monitoring of the at least one control node is at least partially performed by detecting WebSocket events. WebSocket events can be used for monitoring the components of a remote medical imaging control system, possibly in real-time. By establishing a persistent, bi-directional connection between the client and server, WebSockets enable the system to send and receive updates instantly without the need for repetitive polling. For example, various components in a communication system, such as message queues, relay nodes, or network bridges, can emit events indicating their status-such as throughput metrics, error rates, or connection health. These events can be streamed over WebSocket connections to a centralized monitoring dashboard, allowing operators to visualize and react to changes in system performance as they occur.

In practice, the WebSocket server can be programmed to listen to triggers or logs generated by the components of the remote medical imaging control system and relay them to subscribed clients such as the status tracking system. For instance, if a critical node in the communication network experiences a failure or an overload, an event can be pushed to the monitoring client in milliseconds, enabling faster diagnostics and recovery. WebSocket events can also be used to initiate automated responses, such as scaling resources, rerouting traffic, or alerting technical teams. This may provide for more efficient resource usage and may reduce latency in detecting faults or problems.

In another example, the monitoring is performed by any one of the following: executing hardware diagnostics on components of the at least one medical imaging node and/or components of the at least one control node, reading logs that are descriptive of the at least one medical imaging node, reading logs descriptive of the at least one control node, performing log analytics to detect at least one medical imaging node non-standard event, and combinations thereof. For example, a non-standard event may be an event which indicates a fault of failure of a component of the medical imaging system or even of the various medical imaging nodes and control nodes.

In another example, the method further comprises searching the system status database for an inoperable component. The method further comprises performing a corrective protocol if an inoperative component is found in the system status database. The system status database goes through and repeatedly monitors various nodes and updates the system status database in a real time or near real time basis. In the event that a faulty component is detected, for example depending upon the type of fault or the component, a script or other program may be executed which performs the corrective protocol. This for example could be the enabling of a different system to compensate for the failed system in some instances; in other instances this could be sending a message for an operator to repair the defective component. In other examples it could also perform other tasks such as automatically ordering replacements for the inoperable component.

In another example, the corrective protocol comprises any one of the following: sending a repair request for the inoperable component, initializing a reboot of the inoperable component, sending an inoperable component alert to a predetermined control node, and combinations thereof. These actions may all be beneficial because they may help to bring the inoperable component into an operable state as quickly as possible.

In another example, the medical imaging system is any one of the following: a magnetic resonance imaging system, a computed tomography system, a positron emission tomography system, a single photon emission tomography system, a digital fluoroscopy system, and a diagnostic ultrasound system.

In other examples there may be a status tracking system which is used to implement one or more of the method examples above. For example, the status tracking system could be a computer system or computer systems located at one or more locations or may also be virtual machines or real machines that are accessible by the web or the cloud.

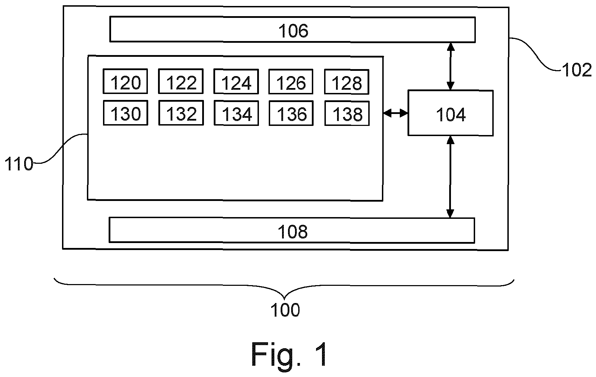

Fig. 1 illustrates an example of a status tracking system 100. The status tracking system 100 is shown as comprising a computer 102. The computer 102 may represent one or more computers or computational systems located at one or more locations. The computer 102 is shown as comprising a processor 104. Likewise, the processor may represent one or more computational systems or processors located at one or more locations. The processor 104 is shown as being connected to a network interface 106 that may be used to communicate with other components of the medical imaging control system. The processor 104 is further shown as optionally being connected to a user interface 108. The user interface may for example enable an operator to modify or control the function of the status tracking system 100. The processor 104 is shown as further being connected to a memory 110. The memory 110 is used to represent various types of memory which may be accessible to the processor 104. In some examples, the memory 110 is a non-transitory storage medium.

The memory 110 is shown as storing machine-executable instructions 120. The machine-executable instructions 120 enable the processor 104 to perform various data analysis and computational tasks. The memory 110 if further shown as containing an operational status received from a medical imaging node 122. This may for example provide for a status of various components such as a KVM system, or various software statuses. The KVM system for example may comprise a screen replicator and/or a control interface. This for example may provide screen replication and also remote human interface device access to a medical imaging node. The operational status 122 of the various components may be stored or updated in a system status database 124. As was mentioned earlier, this may for example be as complicated as a relational database, but it may also be simple and may be a configuration file which stores the status of various components.

The memory 110 is further shown as containing an operational status received from a control node 126. This operational status from the control node 126 is then also used to update the system status database 124. The status from the medical imaging node 122 and the control nodes 126 may be continually updated to provide the system status database 124 accuracy within real time or near real time. The memory 110 is further shown as containing an operational status of a remote medical imaging control system 128. This may for example be the operational status of individual components or parts or it may be an overall status which is provided. The memory 110 is further shown as containing an optional alert signal that was received from a local controller of a medical imaging system.

This for example could be an indication that the operator of the local controller is in need of assistance or an automated system has been triggered to indicate that there is a problem or malfunction with the medical imaging system. The memory 110 is further shown as containing an operational control node 132 that was identified using the system status database 124. This may then cause optional commands 134 to form the network connection between the node-specific control interface that generated the alert signal 130 and the operational control node 132 that was identified in the system status database. This is an example of how the system can be used to provide an automated or real time response from a control node when an alert signal 130 is generated.

The memory 110 is further shown as containing inoperable component 136 that was optionally identified in the system status database 124. This may for example be automatically corrected or compensated for by generating or retrieving a corrective protocol 138 that is appropriate for the inoperable component 136. For example, the corrective protocol 138 may be used to trigger a reboot, send repair commands, or even order a replacement component. As the system status database 124 is updated repeatedly in a periodic fashion, the corrective protocol 138 can be executed more rapidly in an effort to maintain the integrity of the remote medical imaging control system.

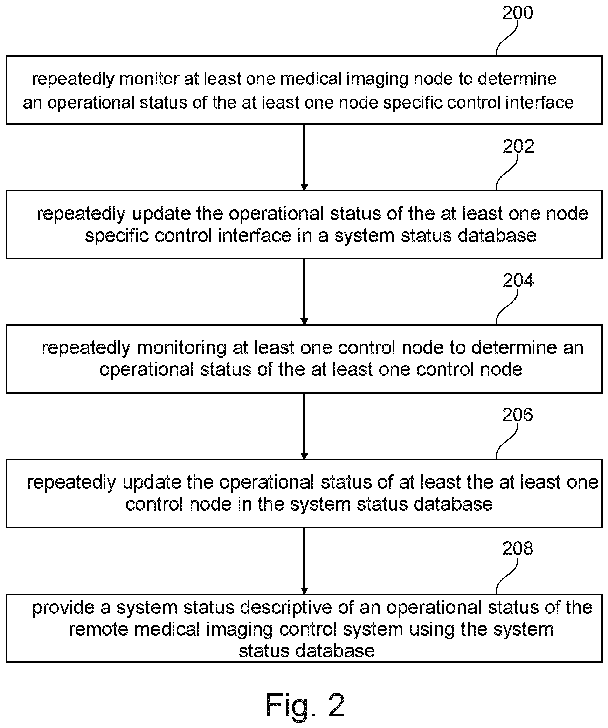

Fig. 2 shows a flowchart which illustrates a method of operating the status tracking system 100 of Fig. 1. In step 200, at least one medical imaging node is repeatedly monitored to determine the operational status 122 of the at least one node-specific control interface. As was mentioned earlier, the at least one medical imaging node comprises a medical imaging system and a local controller. In step 202, the operational status of the at least one node-specific control interface is repeatedly updated in the system status database 124. In step 204, at least one control node is repeatedly monitored to determine an operational status 126 of the at least one control node. In step 206, the operational status 126 of the at least one control node is repeatedly updated in the system status database 124. The steps 200, 202, 204, and 206 may be performed repeatedly to maintain the system status database in a current state. For example, it can be decided that the system status database is maintained to within a certain degree of accuracy in terms of time. In step 208, the system status 128 of the remote medical imaging control system is then provided. Also, as mentioned previously, this may be the status of individual components, it may be a search for inoperable components or for example, it may be presented as a table which summarizes the status of the individual components.

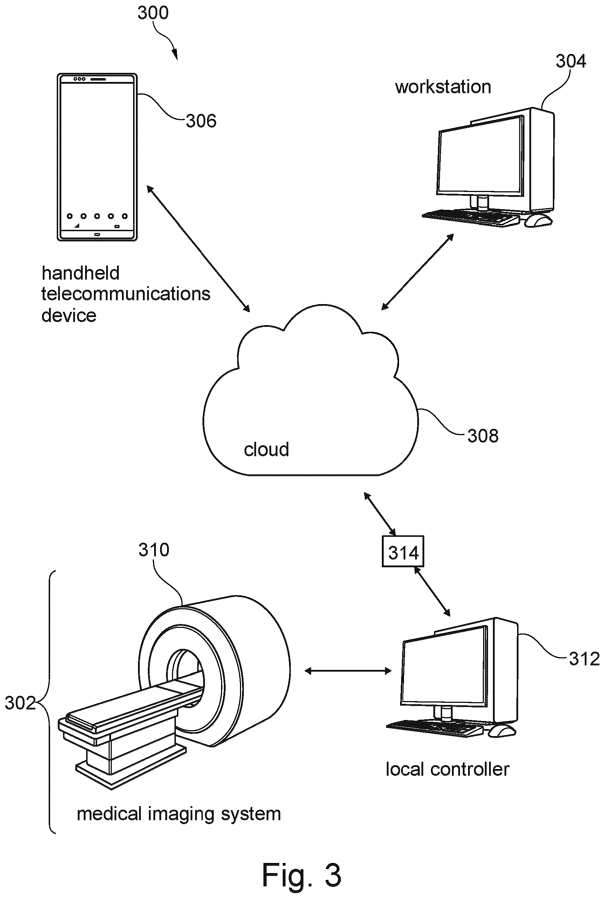

Fig. 3 illustrates an example of a remote medical imaging control system 300. The remote medical imaging control system 300 is shown as comprising a medical imaging node 302 and several control nodes 304, 306. Although only one medical imaging node 302 is shown, there may be multiple medical imaging nodes 302 present.

The control nodes 304, 306 illustrate the different types of computing devices that may be used to function as control nodes. For example, the control node 304 is a workstation or computer type device. The control node 306 is for example a handheld telecommunication device. This may for example be a smartphone or tablet computer as an example.

The medical imaging node 302 is shown as comprising a medical imaging system 310 that is being controlled by a local controller 312. The local controller 312 is controlled by the remote medical imaging control system 300 via a node-specific control interface 314. In some examples this may be a software component installed on the local controller 312 and, for example, enables a remote desktop control of the local controller 312. A particularly advantageous example is when the node-specific control interface 314 is used to replicate a display of the local controller 312 and also provide a remote human interface device like a mouse or keyboard to the local controller 312. The node-specific control interface 314 is shown as being in network connection to the cloud 308 and the two control nodes 304, 306. The functionality of the status tracking system 100 may be distributed in different ways in the remote medical imaging control system 300.

In one example, it could be an additional computer or workstation connected to the cloud 308 and the other nodes 306, 308, 314. In yet another example, the status tracking system 100 is implemented by a server or virtual machine in the cloud 308. In further examples, the functionality of the status tracking system 100 is distributed amongst various components of the remote medical imaging control system 300. For example, some functionality may be implemented themselves within the control nodes 304, 306 or within the cloud 308.

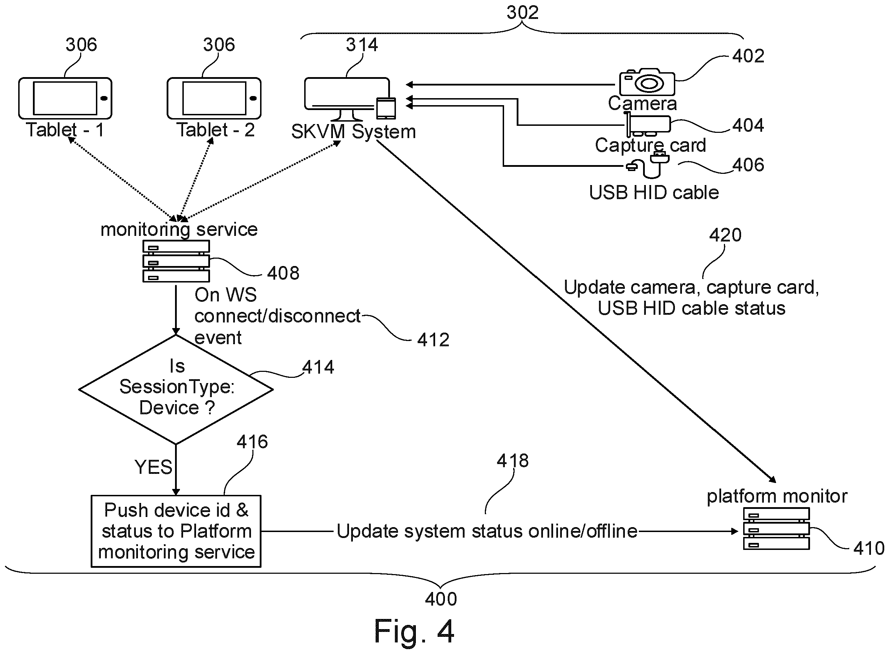

Fig. 4 shows a further example of a remote medical imaging control system 400. Slightly different functionality of the components is explained using this figure. The medical imaging node 302 is shown as comprising the node-specific control interface 314 and having access to a camera 402, a capture card 404, and a USB HID cable 406. The capture card 404 provides replication of the monitor of the local controller 312. The camera 402 may provide example images within the control room of the medical imaging node 302 or views of the subject being imaged by the medical imaging system 310. Some or all of the functionality of the status tracking system 100 is implemented using a monitoring service 408 and a platform monitor 410. The monitoring service 408 monitors the various components 306, 308, 314, 302 of the remote medical imaging control system 400 and the platform monitor 410 hosts the system status database 124. The monitoring service 408 may acts as the intermediary between the devices 306, 314 and the platform monitor 410. It may, for example, listen for WebSocket events that indicate the connection or disconnection of devices (e.g., Tablets, SKVM System). Additionally, it may perform heartbeat checks at fixed intervals to ensure timely updates and maintain device status accuracy. If the monitoring service 408 does not receive a heartbeat response within a specified timeframe, it assumes the connection is lost, indicating that the device is offline.

The monitoring service 408 is shown as being connected to the control nodes 306 and the node-specific control interface 314. When a web socket event showing connect or disconnect is received 412, it is then determined if it is a device 414 or not. For the web sockets, is the session type a device? This indicates that a piece of hardware has been connected or disconnected. If this is the case then, in step 416, the device ID and status is pushed to the platform monitor 410. This is done in step 418 and the system is updated either online or offline. In some cases various components 314 may talk or update the platform monitor 410 directly. In this example, the node-specific control interface 314 is shown as updating the camera capture card and USB cable status 420 in the platform monitor 410 directly.

In some examples, the platform monitor 410 may be responsible for aggregating and storing the status of devices within the remote medical imaging control system 400 (ROCC platform). It could update and maintain the statuses (online/offline/connected/disconnected) for devices and peripherals like cameras, capture cards, and USB HID cables. The platform monitor may have the benefit of ensuring accurate reflection of system availability for customers and internal teams.

The monitored device 302, 306, 314, 402, 404, 406 may include connected devices that send status updates via WebSocket events to the Monitoring Service. A system that includes peripheral devices (camera, capture card, USB HID cable) may be responsible for reporting the status of these peripherals to the monitoring system. Peripherals such as cameras, capture cards, and USB HID cables, whose status (connected/disconnected) is tracked via an SKVM system 314. The system 400 may also use temporary caches device states for quick responses, minimizing frequent data source access.

In the example above various methods may be used for detecting HID Cable 406 and Capture Card 404 status. One method is Windows Management Instrumentation (WMI). WMI is a framework for querying and managing system components. It uses WMI Query Language (WQL) to filter specific events like hardware connections. This method involves using the Windows API to register for device change notifications via RegisterDeviceNotification.

The use of WMI for monitoring may comprise the following sequence:
1. Device Connection/Disconnection:
   - When a Tablet 306 or SKVM 314 system connects/disconnects, a WebSocket event is generated.
   - he monitoring service 408 captures this event and verifies if the session type is a recognized device.
2. Push Status to Platform Monitoring:
   - Once the session type is confirmed, the Monitoring Service pushes the device ID and its online/offline status to the Platform Monitoring Service via an API call.
3. Peripheral Status Updates:
   - The SKVM system 314 monitors peripherals such as cameras 402 and capture cards 404. The status (connected/disconnected) is fetched via the SKVM system and pushed to the Platform Monitoring Service.
4. Platform Monitoring Database:
   - The platform monitor 410 updates the system status database 124 with the new status, ensuring that the current state of all devices and peripherals is accurately stored and reported.

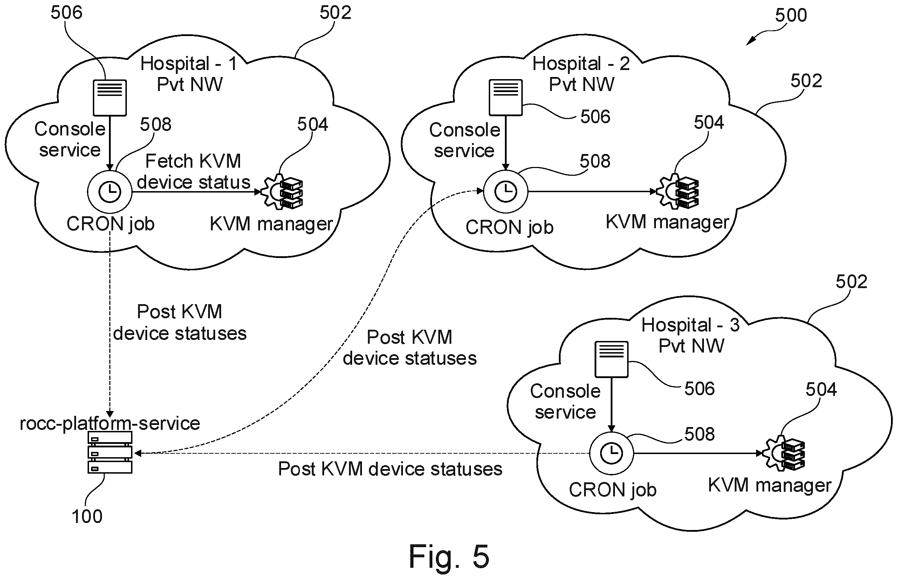

Fig. 5 illustrates a further example of a remote medical imaging control system 500. In this example the status tracking system 100 communicates indirectly with the various medical imaging nodes. The status tracking system 100 is shown as being connected to a number of hospital nodes 502. The hospital nodes represent various locations or groups of where medical imaging systems are installed. These nodes may for example maintain its own local infrastructure.

Within each hospital node 502 is a KVM manager 504 that is used to talk to local node-specific control interfaces any may be responsible for collecting the status data from KVM devices. For example, the node-specific control interfaces may be a KVM system that interfaces with the local controller 312. The KVM manager 504 is able to pull and communicate with a large number of these node-specific control interfaces 314 that are either in one location logically or physically. For example, the hospital zones 502 may indicate different hospitals or may indicate different organizations or groups of hospitals run by the same organization. The KVM manager 504 is then controlled by the console service 506 which is then scheduled by a CRON job 508. The console service 506 may interact with the KVM manager 504 to initiate status updates.

For example, the CRON job 508 may be used to periodically update and control the KVM manager 504 to provide the information on a real time or near real time basis. This could be scheduled by the CRON job to run periodically (e.g., every 5 to 10 minutes, configurable via an environment variable) to fetch the latest KVM device statuses.

In this example, the status update process may include:

### Status Update Process

1. Fetching Device Statuses:
   - The CRON job 508 within the hospital's private network 502 communicates with the KVM manager 504 to fetch updated status information for all connected KVM devices.
   - The statuses include information such as Online/Offline or Connected/Disconnected for each device.
2. Sending Device Status to the status tracking system 100 (ROCC Platform):
   - After retrieving the device statuses, the Console Service posts this information to the centralized status tracking system 100.
   - Each hospital's network operates independently but follows the same process, ensuring that the statuses from multiple hospitals are aggregated on the platform.
3. status tracking system 100:
   - The status tracking system 100 acts as the central repository for device statuses across multiple hospitals.
   - It stores and tracks real-time device states, providing a comprehensive view of all KVM devices and allowing for easy identification of any issues (such as devices going offline).
   - The platform may trigger alerts or notifications based on device statuses to ensure timely action on any detected problems.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

In another aspect of the invention, a non-transitory computer readable medium is described that performs similar steps as described in the method claim and any of the sub-steps described in dependent claims of this invention. In an aspect, a non-transitory computer-readable medium configured for operating a remote medical imaging control system is described, wherein the remote medical imaging control system comprises: at least one medical imaging node (302) comprising a medical imaging system (310) and a local controller (312), wherein the local controller is configured for controlling image acquisition of the medical imaging system, wherein the at least one medical imaging node comprises a node specific control interface (314), wherein the node specific control interface comprises a screen replicator (404) configured to provide a replication of a user interface of the local controller via a network connection, wherein the node specific control interface further comprises at least one input peripheral (406) configured for receiving imaging control signals via the network interface, wherein the imaging control signals are configured for controlling the user interface; and at least one control node (304, 306) configured for connecting to the node specific control interface via the network connection, wherein the at least one control node is further configured to provide the imaging control signals via the network connection in response to receiving the replicated user interface via the network connection. The non-transitory computer-readable medium comprises performing any one of or the combination of: repeatedly monitoring (200) the at least one medical imaging node to determine an operational status (122) of the at least one node specific control interface; repeatedly updating (202) the operational status of the least one node specific control interface in a system status database (124); repeatedly monitoring (204) the at least one control node to determine an operational status (126) of the at least one control node; repeatedly updating (206) the operational status of the at least one control node in the system status database; providing (208) a system status (128) descriptive of an operational status of the remote medical imaging control system using the system status database. Any of the previously described embodiments can be applicable to the computer-readable medium.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: status tracking system
- 102: computer
- 104: processor
- 106: network interface
- 108: optional user interface
- 110: memory
- 120: machine executable instructions
- 122: operational status from medical imaging node
- 124: system status database
- 126: operational status from control node
- 128: system status of remote medical imaging control system
- 130: alert signal from local controller
- 132: operational control node identified in system status database
- 134: commands to form network connection between node specific control interface and operational control node
- 136: inoperable component identified in system status database
- 138: corrective protocol.
- 200: repeatedly monitor at least one medical imaging node to determine an operational status of the at least one node specific control interface
- 202: repeatedly update the operational status of the at least one node specific control interface in a system status database
- 204: repeatedly monitoring at least one control node to determine an operational status of the at least one control node
- 206: repeatedly update the operational status of at least the at least one control node in the system status database
- 208: provide a system status descriptive of an operational status of the remote medical imaging control system using the system status database
- 300: remote medical imaging control system
- 302: medical imaging node
- 304: control node (workstation)
- 306: control node (handheld telecommunication device)
- 308: cloud
- 310: medical imaging system
- 312: local controller
- 314: node specific control interface
- 400: remote medical imaging control system
- 402: camera
- 404: capture card
- 406: USB HID cable
- 408: monitoring service
- 410: platform monitor
- 412: On Web socket connect / disconnect event
- 414: Is session type: Device?
- 416: push device and status to platform monitoring service
- 418: update system status online / offline
- 420: Update camera, capture card, USB HID cable status
- 500: remote medical imaging control system
- 502: hospital zone
- 504: KVM manager
- 506: console service
- 508: CRONjob

## Claims

1. A computer-implemented method of operating a remote medical imaging control system (300, 400, 500), wherein the remote medical imaging control system comprises:
- at least one medical imaging node (302) comprising a medical imaging system (310) and a local controller (312), wherein the local controller is configured for controlling image acquisition of the medical imaging system, wherein the at least one medical imaging node comprises a node specific control interface (314), wherein the node specific control interface comprises a screen replicator (404) configured to provide a replication of a user interface of the local controller via a network connection, wherein the node specific control interface further comprises at least one input peripheral (406) configured for receiving imaging control signals via the network interface, wherein the imaging control signals are configured for controlling the user interface; and
- at least one control node (304, 306) configured for connecting to the node specific control interface via the network connection, wherein the at least one control node is further configured to provide the imaging control signals via the network connection in response to receiving the replicated user interface via the network connection;
wherein the method comprises:
- repeatedly monitoring (200) the at least one medical imaging node to determine an operational status (122) of the at least one node specific control interface;
- repeatedly updating (202) the operational status of the least one node specific control interface in a system status database (124);
- repeatedly monitoring (204) the at least one control node to determine an operational status (126) of the at least one control node;
- repeatedly updating (206) the operational status of the at least one control node in the system status database; and
- providing (208) a system status (128) descriptive of an operational status of the remote medical imaging control system using the system status database.

2. The computer implemented method of claim 1, wherein the method further comprises:
- receiving an alert signal (130) from the local controller of the medical imaging system;
- searching the system status database for an operational control node (132), wherein the at least one control node comprises the operational control node;
- forming the network connection between the operational control node and the at least one medical imaging node;
- sending the replicated user interface from the local controller to the operational control node via the network connection; and
- sending the imaging control signals from the operational control node to the node specific control interface via the network connection in response to receiving the replicated user interface.

3. The computer implemented method of claim 2, wherein the operational control node comprises a robotic process automation system configured to generate the imaging control signals in response to receiving the replicated user interface and the alert signal.

4. The computer implemented method of claim 1, 2, or 3, wherein the remote medical imaging control system further comprises at least one hospital zone (502), wherein the at least one hospital zone comprises multiple of the at least one medical imaging node, wherein the repeated monitoring of the at least one medical imaging node is performed locally within the at least one hospital zone, and wherein the method further comprises polling the at least one hospital zone to update the operational status of at least the at least one medical imaging node in the system status database.

5. The computer implemented method of claim 4, wherein the local monitoring of the at least one medical imaging node is performed using a KVM manager (504) controlled by a console service (506), and wherein the console service is preferably scheduled using a CRON job (508).

6. The computer implemented method of any one of the preceding claims, wherein the node specific control interface is further configured to provide a video feed (402) via the network connection, and wherein the operational status of the node specific control interface is descriptive of an operational status of the video feed, wherein the video feed is any one of the following: a medical imaging node control room video feed, a medical imaging node imaging system room video feed, and combinations thereof.

7. The computer implemented method of any one of the preceding claims, wherein the node specific control interface is further configured to provide a bidirectional intercom channel via the network connection, and wherein the operational status of the node specific control interface is descriptive of an operational status of the bidirectional intercom channel.

8. The computer implemented method of any one of the preceding claims, wherein the remote medical imaging control system comprises a monitoring service (408) configured for monitoring the at least one medical imaging node and the at least one control node and/or wherein the remote medical imaging control system comprises a platform monitor (410) that hosts the system status database.

9. The computer implemented method of any one of the preceding claims, wherein monitoring of the at least one medical imaging node and/or the monitoring of the at least one control node is at least partially performed by detecting WebSocket events.

10. The computer implemented method of any one of the preceding claims, wherein monitoring is performed by any one of the following: executing hardware diagnostics on components of the at least one medical imaging node and/or components of the at least one control node, reading logs descriptive of the at least one medical imaging node, reading logs descriptive of the at least one control node, performing log analytics to detect at least one medical imaging node non-standard event, and combinations thereof.

11. The computer implemented method of any one of the preceding claims, wherein the method further comprises:
- searching the system status database for an inoperable component (136);
- performing a corrective protocol (138) if an inoperable component is found in the system status database.

12. The computer implemented method of claim 11, wherein the corrective protocol comprises any one of the following: sending a repair request for the inoperable component, initiating a reboot of the inoperable component, sending an inoperable component alert to a predetermined control node, and combinations thereof.

13. The computer implemented method of any one of the preceding claims, wherein the medical imaging system is any one of the following: a magnetic resonance imaging system, a computed tomography system, a positron emission tomography system, a single photon emission tomography system, a digital fluoroscopy system, and a diagnostic ultrasound system.

14. A computer program comprising a computer-readable storage medium (110) having machine executable instructions (120) embodied therewith, said machine executable instructions are configured to implement the method of any one of claims 1 through 13.

15. A status tracking system (100) comprising:
- a processor (104); and
- a memory (110) storing machine executable instructions, execution of the machine executable instructions causes the processor to:
- repeatedly monitor (200) at least one medical imaging node (302) to determine an operational status (122) of at least one node specific control interface (314), wherein the at least one medical imaging node comprises a medical imaging system (310) and a local controller (312), wherein the local controller is configured for controlling image acquisition of the medical imaging system, wherein the at least one medical imaging node comprises the node specific control interface, wherein the node specific control interface comprises a screen replicator (404) configured to provide a replication of a user interface of the local controller via a network connection, wherein the node specific control interface further comprises at least one input peripheral configured for receiving imaging control signals via the network interface, wherein the imaging control signals are configured for controlling the user interface;
- repeatedly update (202) the operational status of the at least one node specific control interface in a system status database (124), wherein the at least one control node is configured for connecting to the node specific control interface via the network connection, wherein the at least one control node is further configured to provide the imaging control signals via the network connection in response to receiving the replicated user interface via the network connection;
- repeatedly monitor (204) at least one control node (304, 306) to determine an operational status of the at least one control node;
- repeatedly update (206) the operational status (126) of at least the at least one control node in the system status database; and
- provide (208) a system status (128) descriptive of an operational status of the remote medical imaging control system using the system status database
